# EUROPEAN PATENT APPLICATION

(11) **EP 4 653 414 A1**
(43) Date of publication of application: **26.11.2025**
(21) Application number: 24744333.6
(22) Date of filing: 18.01.2024
(51) Int. Cl.: C07C 13/18, C07D 209/02, C07D 409/14, A61P 29/00, A61K 31/4436

(54) **METHOD FOR PREPARING TETRAHYDRO-1-NAPHTHYLAMINE AND DERIVATIVES THEREOF**

(30) Priority: 18.01.2023 CN 202310084682
(71) Applicant: Shanghai Senhui Medicine Co., Ltd., Shanghai 201203 (CN); Jiangsu Hengrui Pharmaceuticals Co., Ltd., Lianyungang, Jiangsu 222047 (CN)
(72) Inventor: JIANG, Wei, Shanghai 201203 (CN); HUANG, Qing, Shanghai 201203 (CN); GAO, Junfeng, Shanghai 201203 (CN); HUANG, Jian, Shanghai 201203 (CN)
(74) Representative: Potter Clarkson
(86) International application number: PCT/CN2024/072912
(87) International publication number: WO 2024/153159

(57) **Abstract**

The present disclosure relates to a method for preparing tetrahydro-1-naphthylamine and derivatives thereof. Specifically, the present disclosure relates to a method for preparing (1S,4S)-4-methoxy-1,2,3,4-tetrahydronaphthalen-1-amine, and the method comprises the step of reacting compound C-1 under the condition of (R)-2-methyl-CBS-oxazaborolidine so as to form compound D-1. The method is simple to operate and is suitable for industrial production.

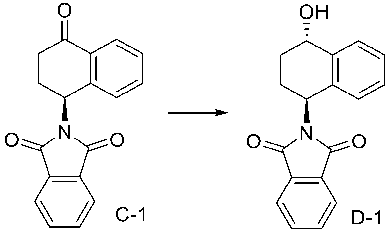

## Description

### TECHNICAL FIELD

The present disclosure pertains to the field of pharmaceutics, and relates to a method for preparing tetrahydro-1-naphthylamine and a derivative thereof.

### BACKGROUND

Chiral amines are components or fragments of numerous compound structures with important biological activity. For example, (1*S*,4*S*)-4-methoxy-1,2,3,4-tetrahydronaphthalen-1-amine is a key intermediate for the synthesis of the MOR agonist (1*S*,4*S*)-4-ethoxy-*N*-(2-((*R*)-9-(pyridin-2-yl)-6-oxaspiro[4.5]decan-9-yl)ethyl)-1,2,3,4-tetrahydronaphthalen-1-amine.

WO2017063509 disclosed a method in which (*S*)-4-carbonyl-1,2,3,4-tetrahydronaphthalene-1-carbamate is used as a starting material and subjected to a reaction in the presence of a chiral reductant to give tert-butyl (1*S*, 4*S*)-4-hydroxy-1,2,3,4-tetrahydronaphthalene-1-carbamate, and the product is then subjected to etherification and deprotection to give the target product:

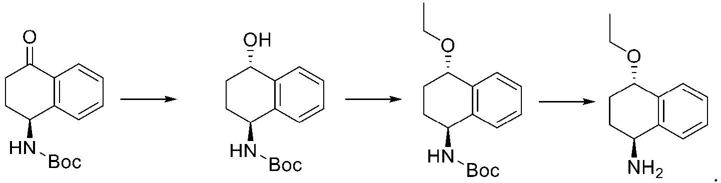

### SUMMARY

The present disclosure provides a method for preparing a compound represented by formula I or a salt thereof: 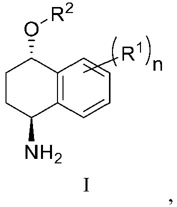 comprising a step of reacting a compound represented by formula C in the presence of (*R*)-2-methyl-CBS-oxazaborolidine to form a compound represented by formula D, 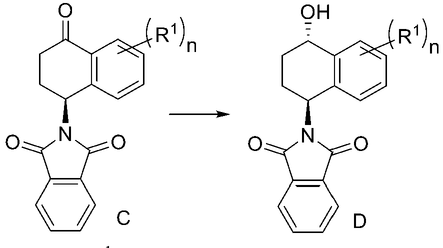 wherein R¹ is selected from the group consisting of halogen, nitro, cyano, C₁₋₆ alkyl, and C₁₋₆ alkoxy, and the alkyl or alkoxy is optionally substituted with one or more groups selected from the group consisting of halogen, oxo, nitro, cyano, C₁₋₆ alkyl, and C₁₋₆ alkoxy; n is 0, 1, 2, or 3; R² is selected from the group consisting of hydrogen, C₁₋₆ alkyl, and C₃₋₆ cycloalkyl, and the alkyl or cycloalkyl is optionally substituted with one or more groups selected from the group consisting of halogen, oxo, nitro, cyano, C₁₋₆ alkyl, C₁₋₆ alkoxy, and C₃₋₆ cycloalkyl.

In some embodiments, in the method, the molar amount ratio of (*R*)-2-methyl-CBS-oxazaborolidine to the compound represented by formula C is 1:10 to 1:1, including but not limited to 1:10, 1:9, 1:8, 1:7, 1:6, 1:5, 1:4, 1:3, 1:2, 1:1, or a value between any two of these numbers. In some embodiments, in the method, the molar amount ratio of (*R*)-2-methyl-CBS-oxazaborolidine to the compound represented by formula C is 1:10.

In some embodiments, the process of reacting the compound represented by formula C to form the compound represented by formula D also involves a reductant, and the reductant is selected from the group consisting of, but is not limited to, borane. In some embodiments, in the method, the amount of borane used is 0.5-1 equivalent (eq.) of the molar amount of the compound represented by formula C, including but not limited to 0.5 equivalents, 0.6 equivalents, 0.7 equivalents, 0.8 equivalents, 0.9 equivalents, 1.0 equivalent, or a value between any two of these numbers. In some embodiments, in the method, the amount of borane used is 0.8-1 equivalent (eq.) of the molar amount of the compound represented by formula C.

In some other embodiments, the solvent used in the reaction of the compound represented by formula C is selected from the group consisting of, but is not limited to, toluene and tetrahydrofuran. In some embodiments, the solvent used in the reaction of the compound represented by formula C is tetrahydrofuran.

In another aspect, the temperature of the reaction of the compound represented by formula C is -10 to 10 °C, including but not limited to -10°C, -8 °C, -6 °C, -4 °C, -2 °C, 0 °C, 2 °C, 4 °C, 6 °C, 8 °C, 10°C, or a value between any two of these numbers. Further, the method for preparing the compound represented by formula I or the salt thereof provided in some embodiments further comprises reacting a compound represented by formula B in the presence of an oxidant to form the compound represented by formula C: 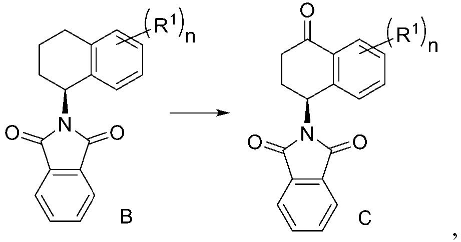 wherein R¹ and n are as defined previously.

In some embodiments, the oxidant is selected from the group consisting of, but is not limited to, potassium permanganate and sodium hypochlorite. In some embodiments, in the method, the compound represented by formula B is reacted in the presence of potassium permanganate to form the compound represented by formula C. In some embodiments, the temperature of the reaction of the compound represented by formula B is 0-30 °C, including but not limited to 0 °C, 6 °C, 10 °C, 16 °C, 20 °C, 26 °C, 30 °C, or a value between any two of these numbers.

In some embodiments, the solvent used in the reaction of the compound represented by formula B is acetone. In some other embodiments, in the method, the amount of the oxidant used is 1-6 equivalents (eq.) of the molar amount of the compound represented by formula B, including but not limited to 1 equivalent, 2 equivalents, 3 equivalents, 4 equivalents, 5 equivalents, 6 equivalents, or a value between any two of these numbers. The method for preparing the compound represented by formula I or the salt thereof provided in some other embodiments further comprises a step of reacting a compound represented by formula A with phthalic anhydride to form the compound represented by formula B: 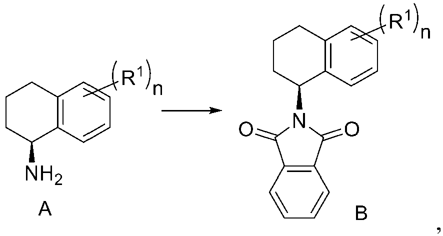 wherein R¹ and n are as defined previously.

In some embodiments, in the method, the molar amount ratio of the compound represented by formula A to phthalic anhydride is 1:1 to 1:3, including but not limited to 1:1, 1:1.2, 1:1.4, 1:1.6, 1:1.8, 1:2, 1:2.2, 1:2.4, 1:2.6, 1:2.8, or a value between any two of these numbers. In some embodiments, in the method, the molar amount ratio of the compound represented by formula A to phthalic anhydride is 1:1.

In some other embodiments, the solvent used in the reaction of the compound represented by formula A is toluene.

In some embodiments, the method comprises the following steps:
a) reacting the compound represented by formula A with phthalic anhydride to form the compound represented by formula B,
b) reacting the compound represented by formula B in the presence of the oxidant to form the compound represented by formula C, and
c) reacting the compound represented by formula C in the presence of (R)-2-methyl-CBS-oxazaborolidine to form the compound represented by formula D: 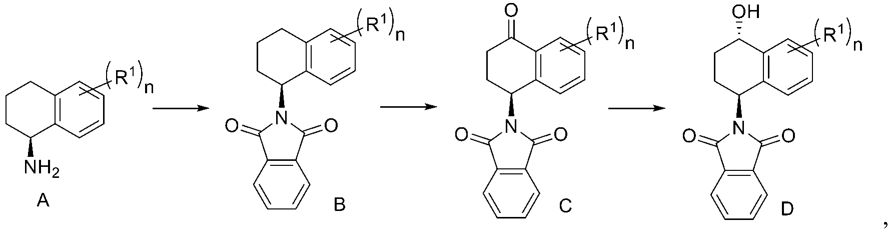
wherein R¹ and n are as defined previously.

In some other embodiments, in the method, the compound represented by formula I is a compound represented by formula I-1: 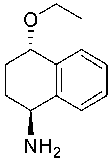 ^{I-1} , and the method comprises a step of reacting compound C-1 in the presence of (R)-2-methyl-CBS-oxazaborolidine to form compound D-1:

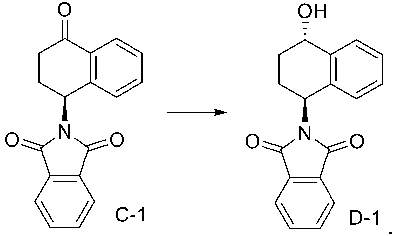

In some embodiments, the method for preparing the compound represented by formula I-1 comprises a step of reacting compound B-1 in the presence of an oxidant, such as potassium permanganate to form compound C-1:

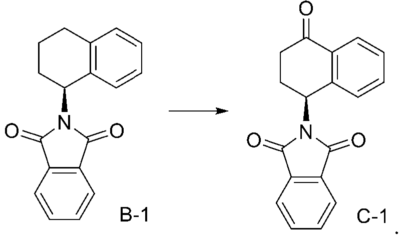

In some other embodiments, the method for preparing the compound represented by formula I-1 comprises a step of reacting compound A-1 in the presence of phthalic anhydride to form compound B-1:

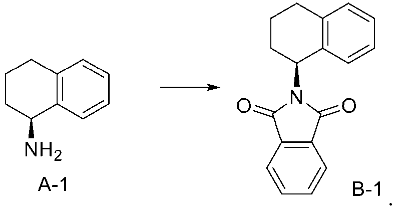

In some embodiments, the method for preparing the compound represented by formula I-1 comprises the following steps:
a) reacting compound A-1 with phthalic anhydride to form compound B-1,
b) reacting compound B-1 in the presence of an oxidant to form compound C-1, and
c) reacting compound C-1 in the presence of (R)-2-methyl-CBS-oxazaborolidine to form compound D-1:

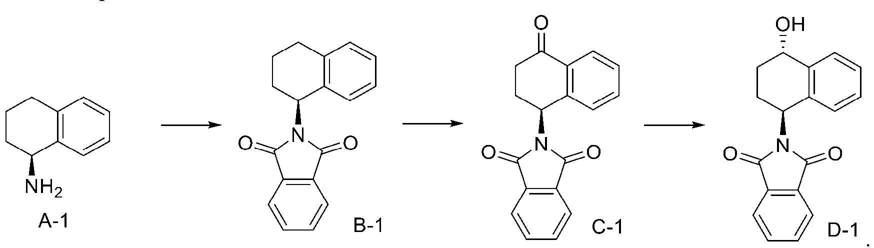

In some embodiments, in step a), compound A-1 is reacted with phthalic anhydride under heating conditions to form compound B-1.

In some embodiments, in step a), compound A-1 is reacted with phthalic anhydride in the presence of triethylamine to form compound B-1.

In some embodiments, in step b), compound B-1 is reacted in the presence of potassium permanganate to form compound C-1.

In some embodiments, in step c), compound C-1 is reduced in the presence of (R)-2-methyl-CBS-oxazaborolidine to form compound D-1. In some embodiments, the reductant used in the reduction of compound C-1 is borane.

Further, the method for preparing the compound represented by formula I-1 further comprises a step of reacting compound D-1 with halogenated ethane to form a compound represented by formula E-1, and subsequently removing a protecting group from compound E-1 to form the compound of formula I-1 or the salt thereof:

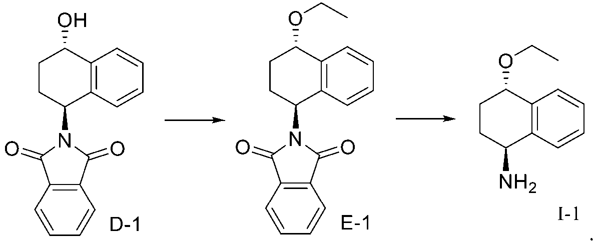

In some embodiments, in the method, the halogenated ethane is selected from the group consisting of iodoethane and bromoethane. In some other embodiments, compound D-1 is reacted with halogenated ethane such as iodoethane in the presence of silver oxide to form compound E-1.

In some embodiments, compound E-1 is reacted in the presence of methylamine to form the compound of formula I-1. For relevant experimental procedures, reference can be made to Protective Groups in Organic Synthesis, 5Th Ed. T. W. Greene & P. G. M. Wuts, and relevant contents are incorporated herein for illustrative purposes.

The present disclosure further provides a compound represented by formula D or a pharmaceutically acceptable salt thereof: 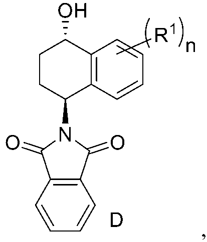 wherein R¹ is selected from the group consisting of halogen, nitro, cyano, C₁₋₆ alkyl, and C₁₋₆ alkoxy, and the alkyl or alkoxy is optionally substituted with one or more groups selected from the group consisting of halogen, oxo, nitro, cyano, C₁₋₆ alkyl, and C₁₋₆ alkoxy; n is 0, 1, 2, or 3.

In some embodiments, the compound represented by formula D is compound D-1:

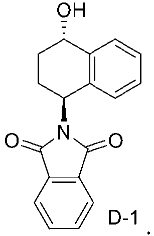

The present disclosure further provides a compound represented by formula C or a pharmaceutically acceptable salt thereof: 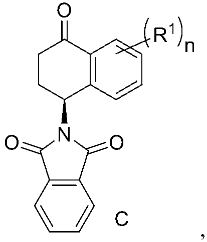 wherein R¹ is selected from the group consisting of halogen, nitro, cyano, C₁₋₆ alkyl, and C₁₋₆ alkoxy, and the alkyl or alkoxy is optionally substituted with one or more groups selected from the group consisting of halogen, oxo, nitro, cyano, C₁₋₆ alkyl, and C₁₋₆ alkoxy; n is 0, 1, 2, or 3.

In some embodiments, the compound represented by formula C is compound C-1:

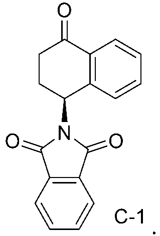

The present disclosure further provides a method for preparing compound AA, comprising the aforementioned steps of preparing the compound represented by formula I:

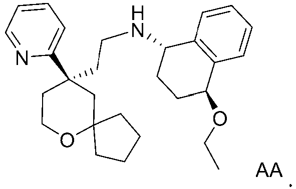

In some embodiments, the method for preparing compound AA further comprises a step of reacting the compound of formula I-1 with compound F to form compound AA: 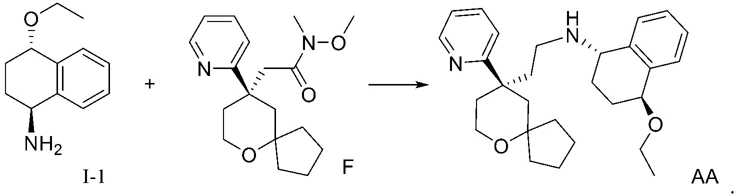 For relevant experimental procedures, reference can be made to WO2019062804, and relevant contents are incorporated herein for illustrative purposes.

In another aspect, the preparation methods of the present disclosure further comprise one or more steps of filtration, concentration, column chromatography purification, and drying.

In the present disclosure, the terms "form" and "convert into" do not specifically refer to a single-step conversion reaction between two substrates and may refer to a single-step or multi-step reaction between the two substrates. If an intermediate contains a protecting group, the intermediate is subjected to one-step removal of the protecting group and then reacted with the corresponding substrate to give the corresponding target product.

The numerical values in the present disclosure are instrument measurements and have a certain degree of error. Generally, ±10% is a reasonable margin of error. It is certainly necessary to consider the context in which the numerical values are used; for example, the numerical value of the particle size of the active ingredient after measurement has a margin of error of no greater than ±10%; the margin of error may be ±9%, ±8%, ±7%, ±6%, ±5%, ±4%, ±3%, ±2%, or ±1%, preferably ±5%.

The pharmaceutically acceptable salts of the compounds described in the present disclosure may be selected from the group consisting of inorganic salts and organic salts, including "acid" addition salts and "base" addition salts. For example, salts formed by acid-base reactions with basic groups (amino groups) are included, and the acid includes organic or inorganic acids. In some embodiments, the salt of the compound represented by formula I is an oxalate.

In the chemical structures of the compounds of the present disclosure, the bond " 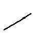 " indicates an unspecified configuration; that is, if chiral isomers exist in the chemical structures, the bond " 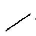 " may be 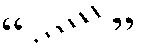 or 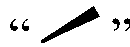 , or includes both the configurations 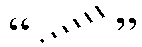 and 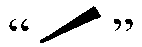 simultaneously.

The term "alkyl" refers to a saturated aliphatic hydrocarbon group, including alkyl groups having 1 to 6 carbon atoms. Non-limiting examples include methyl, ethyl, n-propyl, and isopropyl. Alkyl may be optionally substituted or unsubstituted.

The term "alkoxy" refers to -O-(alkyl), wherein the alkyl is as defined above. Non-limiting examples of alkoxy include: methoxy, ethoxy, propoxy, and butoxy. Alkoxy may be optionally substituted or unsubstituted.

The term "cycloalkyl" refers to a saturated or partially unsaturated, monocyclic or polycyclic hydrocarbon substituent; the cycloalkyl ring contains 3 to 6 carbon atoms. Non-limiting examples include cyclopropyl, cyclobutyl, cyclopentyl, cyclopentenyl, cyclohexyl, cyclohexenyl, cyclohexadienyl, and the like. Cycloalkyl may be substituted or unsubstituted, and when it is substituted, the substituent may be substituted at any accessible point of attachment.

The term "halogen" refers to fluorine, chlorine, bromine, or iodine.

The term "cyano" refers to -CN.

The term "amino" refers to -NH₂.

The term "nitro" refers to -NO₂.

The term "oxo" refers to the =O substituent.

When a functional group of the present disclosure is substituted, the substituent is preferably one or more of the following groups, such as halogen, oxo, nitro, cyano, C₁₋₆ alkyl, C₁₋₆ alkoxy, or C₃₋₆ cycloalkyl.

### DETAILED DESCRIPTION

The present disclosure is further described below with reference to examples. However, these examples do not limit the scope of the present disclosure.

Experimental methods without specific conditions indicated in the examples of the present disclosure were generally conducted under conventional conditions, or conditions recommended by the manufacturers of the starting materials or commercial products. Reagents without specific sources indicated were commercially available conventional reagents.

The structures of the compounds were determined by nuclear magnetic resonance (NMR) spectroscopy or/and mass spectrometry (MS). The NMR shifts (δ) are given in 10-6 (ppm).

The NMR analyses were performed using a Bruker AVANCE-400 nuclear magnetic resonance instrument, with chloroform-D (CDCl₃) as a solvent.

The MS analyses were performed using a Waters Micromass Quattro micro API triple quadrupole mass spectrometer in positive/negative ion scan mode, with the mass scan range being 120-1300.

HPLC columns: YMC-Pack ODS-A (3 µm, 4.6 mm × 150 mm)

The thin-layer chromatography silica gel plates used were Yantai Huanghai HSGF254 silica gel plates. The silica gel plates used in the thin-layer chromatography (TLC) analyses had a layer thickness of 0.2 mm ± 0.03 mm, and those used in the thin-layer chromatography separation and purification had a layer thickness of 0.4 mm-0.5 mm.

### Example 1:

### Step 1) Preparation of (S)-2-(1,2,3,4-tetrahydronaphthalen-1-yl)isoindoline-1,3-dione

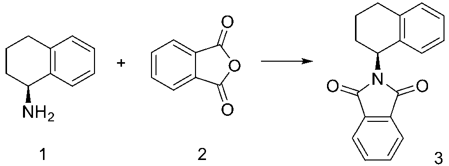

Starting material **1** (100.0 g, 1.0 eq.) and phthalic anhydride (100.0 g, 1.0 eq.) were added to a 1 L flask, heated to about 140 °C, and reacted at that temperature. After the reaction was complete as confirmed by HPLC analysis, the mixture was slowly cooled. Crystallization was performed using isopropanol (600 mL, 6.0 V). After filtration and drying, an off-white solid (166.0 g, yield: 88.3%, purity: 98.8%) was obtained.

¹HNMR (400 MHz, DMSO-d₆): δ 7.84-7.89 (t., 4H), 7.12-7.19 (m, 2H), 7.01-7.06 (m, 1H), 6.93-6.94 (d, 1H), 5.36-5.40 (m, 1H), 2.80-2.87 (m, 2H), 2.24-2.30 (m, 1H), 2.02-2.05 (m, 2H), 1.79-1.83 (m, 1H).

LCMS (ESI): m/z 278[M+1]⁺.

### Step 2) Preparation of (S)-2-(4-oxo-1,2,3,4-tetrahydronaphthalen-1-yl)isoindoline-1,3-dione

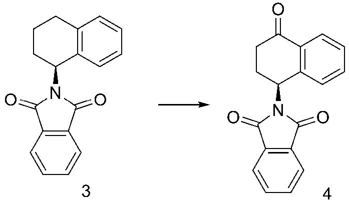

Compound **3** (60.0 g, 1.0 eq.), water (600 mL, 10.0 V), acetone (1200 mL, 20.0 V), and magnesium sulfate (52.1 g, 2.0 eq.) were added to a 5 L flask, and potassium permanganate (170.6 g, 5.0 eq.) was added at 0-10 °C. After the addition, the mixture was naturally warmed to room temperature and reacted overnight. After the reaction was complete as confirmed by HPLC analysis, EA (1000 mL, 16.7 V) was added, and water (1000 mL, 16.7 V) was added. The reaction was quenched with a saturated aqueous solution of sodium thiosulfate in an ice bath. The reaction mixture was filtered, and the filter cake was rinsed with EA. The resulting filtrate was subjected to liquid separation. The organic phase was washed with 1000 mL of water once, dried over anhydrous sodium sulfate, filtered, and concentrated to give a crude product. EA (180 mL, 3.0 V) and petroleum ether (540 mL, 9.0 V) were added to the crude product, and slurrying was performed. After filtration and drying under vacuum, product **4** was obtained as an off-white solid (48.5 g, yield: 77.0%, purity: 98.4%).

¹HNMR (400 MHz, DMSO-d₆): δ 7.87-7.95 (t., 5H), 7.51-7.55 (m, 1H), 7.41-7.45 (m, 1H), 7.24-7.26 (d, 1H), 5.71-5.75 (m, 1H), 2.91-2.95 (m, 1H), 2.71-2.76 (m, 2H), 2.28-2.30 (m, 1H).

LCMS (ESI): m/z 292[M+1]⁺.

### Step 3) Preparation of 2-((1S,4S)-4-hydroxy-1,2,3,4-tetrahydronaphthalen-1-yl) isoindoline-1,3-dione

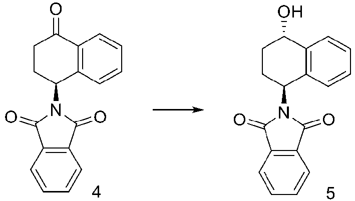

Compound **4** (95.0 g, 1.0 eq.), (*R*)-2-methyl-CBS-oxazaborolidine (9.0 g, 0.1 eq.), and THF (475.0 mL, 5.0 V) were added to a 1 L reaction flask, and the mixture was stirred for complete dissolution. In an ice bath, 1 M borane/tetrahydrofuran (176 mL, 0.54 eq.) was added dropwise. After the addition, the mixture was reacted at about 0 °C. After the reaction was complete as confirmed by HPLC analysis, 20 mL of acetic acid was slowly added dropwise to quench the reaction. EA (500 mL) and water (500 mL) were added for extraction, and liquid separation was performed. The aqueous phase was back-extracted with EA (300 mL). The organic phases were combined, washed with 300 mL of water once, and then washed with 300 mL of 5% sodium bicarbonate once, and liquid separation was performed. The organic phase was dried over anhydrous sodium sulfate. After filtration and concentration, product **5** was obtained as an off-white solid (93.7 g, yield: 97.9%). According to HPLC analysis, 93.6% of the solid was the product (2.6% of the solid was a diastereomer).

¹HNMR (400 MHz, DMSO-d₆): δ 7.86-7.89 (m, 4H), 7.54-7.56 (d, 1H), 7.20-7.24 (t, 1H), 7.08-7.11 (t, 1H), 6.91-6.93 (d, 1H), 5.38-5.42 (m, 2H), 4.70-4.73 (m, 1H), 2.31-2.35 (m, 1H), 2.19-2.23 (m, 1H), 2.06-2.19 (m, 1H), 1.71-1.75 (m, 1H).

LCMS (ESI): m/z 276[M-17]⁺ (EM = 293).

### Step 4) Preparation of 2-((1S,4S)-4-ethoxy-1,2,3,4-tetrahydronaphthalen-1-yl) isoindoline-1,3-dione

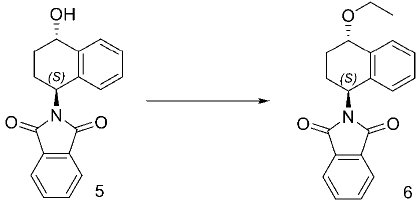

Compound **5** (83.27 g, 1.0 eq.), silver oxide (131.6 g, 2.0 eq.), 4A powdered molecular sieve (249.8 g, 3.0 wt), tetrabutylammonium iodide (209.7 g, 2.0 eq.), and dichloromethane (832 mL, 10.0 V) were sequentially added to a reaction flask, and iodoethane (398.5 g, 9.0 eq.) was finally added. The external temperature was raised to 40 °C for reaction. When the content of the starting material was less than 1.0% as confirmed by HPLC in-process control, the mixture was cooled to room temperature and filtered through diatomaceous earth, and the filter cake was rinsed with dichloromethane (249 mL, 3 V). The filtrate was concentrated to dryness. Ethyl acetate (832 mL, 10 V) was added, and slurrying was performed at room temperature. After filtration, the filter cake was rinsed with ethyl acetate (249 mL, 3 V), and the filtrate was washed with purified water (416 mL × 2, 5 V × 2) twice. The organic phase was dried over anhydrous sodium sulfate, filtered, and concentrated to dryness to give a crude product (92.8 g, purity: 93.6%), and the crude product was directly used in the next step.

¹HNMR (400 MHz, CDCl₃): δ 7.81-7.85 (m, 2H), 7.71-7.75 (m, 2H), 7.55-7.57 (d, 1H), 7.23-7.26 (t, 1H), 7.11-7.15 (t, 1H), 6.92-6.94 (d, 1H), 5.56-5.60 (m, 1H), 4.69-4.72 (m, 1H), 3.71-3.80 (m, 2H), 2.36-2.46 (m, 2H), 2.21-2.26 (m, 1H), 1.85-1.88 (m, 1H), 1.30-1.33 (t, 3H).

LCMS (ESI): m/z 276[M-45]⁺ (EM = 321).

### Step 5) Preparation of (1S,4S)-4-ethoxy-1,2,3,4-tetrahydro-1-naphthylamine

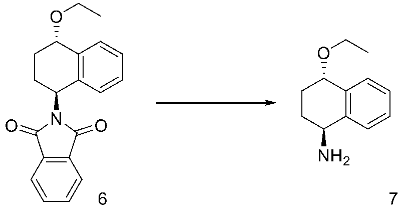

Compound 6 (80.0 g) and methanol (640 mL, 8 V) were added to a 1 L three-necked flask, and the mixture was heated to 50 °C and stirred for dissolution. A 30% aqueous solution of methylamine (411.5 g) was added to the solution. After the mixture was stirred at an internal temperature of 45-55 °C for 20-24 h, the reaction was complete. The reaction mixture was cooled to room temperature and filtered through diatomaceous earth, and the filtrate was concentrated under reduced pressure to give a crude product. After column chromatography purification, compound 7 was obtained as an off-white solid (37.7 g, HPLC purity: 98.6%, chiral purity: 99.7%, two-step yield: 80.6%).

1H NMR (400 MHz, DMSO-d₆): δ 7.46 (d, J = 7.34 Hz, 1H), 7.29 (d, J = 7.34 Hz, 1H), 7.12-7.26 (m, 2H), 4.37 (s, 1H), 3.83 (s, 1H), 3.60 (d, J = 6.85 Hz, 1H), 3.48 (d, J = 6.85 Hz, 1H), 2.01-2.19 (m, 2H), 1.87 (br, 2H), 1.65-1.75 (m, 1H), 1.39-1.52 (m, 1H), 1.14 (d, J = 6.97 Hz, 3H).

LCMS (ESI): m/z 192[M+H]⁺.

## Claims

1. A method for preparing a compound represented by formula I or a salt thereof: 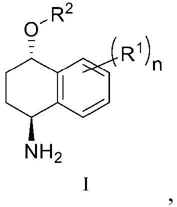 comprising a step of reacting a compound represented by formula C in the presence of (R)-2-methyl-CBS-oxazaborolidine to form a compound represented by formula D, 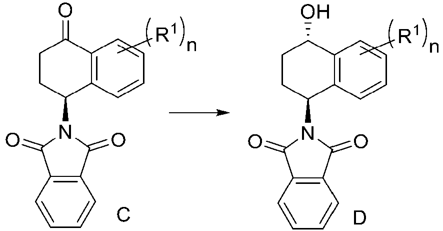 wherein R¹ is selected from the group consisting of halogen, nitro, cyano, C₁₋₆ alkyl, and C₁₋₆ alkoxy, and the alkyl or alkoxy is optionally substituted with one or more groups selected from the group consisting of halogen, oxo, nitro, cyano, C₁₋₆ alkyl, and C₁₋₆ alkoxy; n is 0, 1, 2, or 3; R² is selected from the group consisting of hydrogen, C₁₋₆ alkyl, and C₃₋₆ cycloalkyl, and the alkyl or cycloalkyl is optionally substituted with one or more groups selected from the group consisting of halogen, oxo, nitro, cyano, C₁₋₆ alkyl, C₁₋₆ alkoxy, and C₃₋₆ cycloalkyl.

2. The method according to claim 1, wherein the molar amount ratio of (R)-2-methyl-CBS-oxazaborolidine to the compound represented by formula C is 1:10 to 1:1, preferably 1:10.

3. The method according to claim 1 or 2, further comprising reacting a compound represented by formula B in the presence of an oxidant to form the compound represented by formula C: 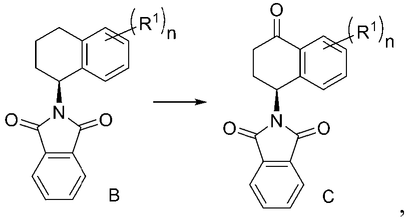 wherein R¹ and n are as defined in claim 1.

4. The method according to claim 3, wherein the oxidant is selected from the group consisting of potassium permanganate and sodium hypochlorite.

5. The method according to any one of claims 1-4, further comprising a step of reacting a compound represented by formula A with phthalic anhydride to form the compound represented by formula B: 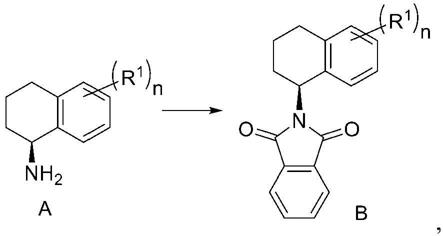 wherein R¹ and n are as defined in claim 1.

6. The method according to any one of claims 1-5, comprising the following steps:
a) reacting the compound represented by formula A with phthalic anhydride to form the compound represented by formula B,
b) reacting the compound represented by formula B in the presence of the oxidant to form the compound represented by formula C, and
c) reacting the compound represented by formula C in the presence of (R)-2-methyl-CBS-oxazaborolidine to form the compound represented by formula D: 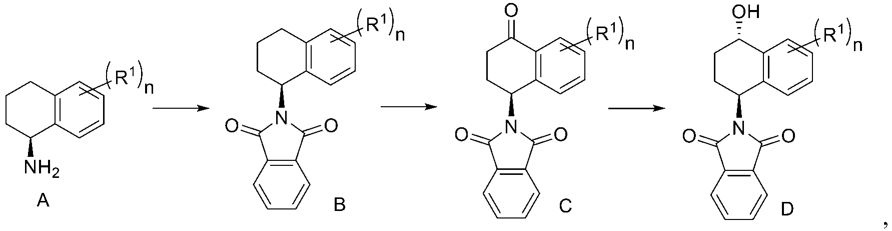 wherein R¹ and n are as defined in claim 1.

7. The method according to any one of claims 1-6, wherein the compound represented by formula I is a compound represented by formula I-1: 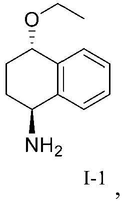 and the method comprises a step of reacting compound C-1 in the presence of (R)-2-methyl-CBS-oxazaborolidine to form compound D-1:

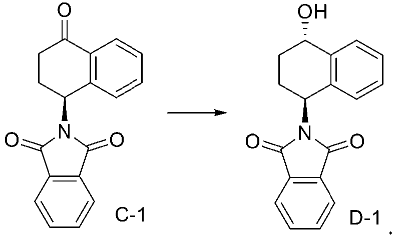

8. The method according to claim 7, further comprising a step of reacting compound D-1 with halogenated ethane to form compound E-1 and subsequently removing a protecting group from compound E-1 to form the compound of formula I-1:

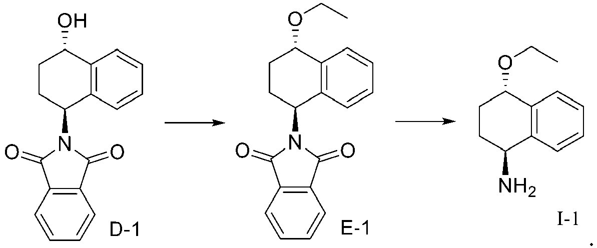

9. A compound represented by formula D or a pharmaceutically acceptable salt thereof: 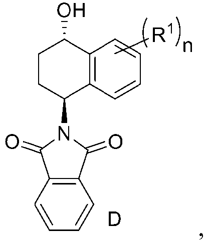 wherein R¹ is selected from the group consisting of halogen, nitro, cyano, C₁₋₆ alkyl, and C₁₋₆ alkoxy, and the alkyl or alkoxy is optionally substituted with one or more groups selected from the group consisting of halogen, oxo, nitro, cyano, C₁₋₆ alkyl, and C₁₋₆ alkoxy; n is 0, 1, 2, or 3.

10. A compound represented by formula C or a pharmaceutically acceptable salt thereof: 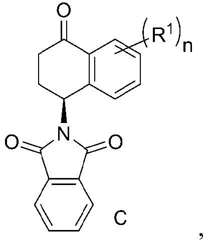 wherein R¹ is selected from the group consisting of halogen, nitro, cyano, C₁₋₆ alkyl, and C₁₋₆ alkoxy, and the alkyl or alkoxy is optionally substituted with one or more groups selected from the group consisting of halogen, oxo, nitro, cyano, C₁₋₆ alkyl, and C₁₋₆ alkoxy; n is 0, 1, 2, or 3.

11. A method for preparing compound AA, comprising the step(s) of the method according to any one of claims 1-8:
